(19) Europäisches Patentamt
European Patent Office
Office européen des brevets

(11) Veröffentlichungsnummer: **0 019 304**
**B1**

(12) # EUROPÄISCHE PATENTSCHRIFT

(45) Veröffentlichungstag der Patentschrift: **15.12.82**

(51) Int. Cl.³: **C 07 C 69/157**, C 07 C 67/00// C07F5/00

(21) Anmeldenummer: **80200077.8**

(22) Anmeldetag: **29.01.80**

(54) **Verfahren zur Herstellung der Arylester von Carbonsäuren.**

(30) Priorität: **09.05.79 DE 2918592**

(43) Veröffentlichungstag der Anmeldung: **26.11.80 (Patentblatt 80/24)**

(45) Bekanntmachung des Hinweises auf die Patenterteilung: **15.12.82 Patentblatt 82/50**

(84) Benannte Vertragsstaaten: **DE FR GB IT NL**

(56) Entgegenhaltungen:
**US A 4 182 915**
**THE JOURNAL OF THE AMERICAN CHEMICAL SOCIETY, Band 92, 1970, E.C. TAYLOR et al.: « Thallium in organic synthesis. XV. Synthesis of Phenols and Aromatic Nitriles », Seiten 3 520-3 522.**

(73) Patentinhaber: **Rütgerswerke Aktiengesellschaft**
**Mainzer Landstrasse 217**
**D-6000 Frankfurt a.Main 1 (DE)**

(72) Erfinder: **Knips, Ulrich, Dr.**
**Im Baumhof 1**
**D-5970 Plettenberg (DE)**

**EP 0 019 304 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung der Arylester von Carbonsäuren

Die Erfindung betrifft ein Verfahren zur Herstellung isomerenreiner Carbonsäurearylester durch katalytische Zersetzung der entsprechenden Monoarylthalliumsalze unter Erhaltung des Substitutionsmusters der Arylrestes nach Art einer Dethallierungsreaktion.

Durch Umsetzung von aromatischen Kohlenwasserstoffen mit Thallium (III) salzen sind Monoarylthalliumsalze in hoher Isomerenreinheit erhältlich, wobei das Anion des Thalliumsalzes sowohl anorganischer als auch organischer Natur sein kann. Die Reaktion von Arylthalliumbis (trifluoracetaten) mit Blei (IV)-acetat ist in J. Am. Soc. 92.3520 (1970) beschrieben. Dabei treten als Zwischenstufen die Trifluoressigsäurearylester auf. Der Arylester selbst wurde danach nicht isoliert, sondern das entsprechende Phenol. Die vorgenannte Methode eignet sich wegen der großen Menge an Hilfsstoffen nicht für die technische Herstellung von Arylestern. Ferner ist es aus Chem. Comm. 390 (1971) bekannt, salzartige Arylthalliumverbindungen mit Palladiumchlorid umzusetzen. Als Reaktionsmedium war konz. Essigsäure angegeben. Als Produkte wurden bei diesem Verfahren Chloraromaten und überwiegend Biphenyle erhalten.

Die US-PS, 4,182,915 beschreibt ein Verfahren zur Herstellung nucleophil ringsubstituierter aromatischer Verbindungen durch Umsetzung eines Aryl-thallium (III)-salzes mit einem nucleophilen Reagens. Ist der nucleophile Substituent $RCOO^-$, der in Form der Carbonsäure zugegeben wird, so soll der entsprechende Arylester entstehen. Im wässrigen Medium reagiert unter den gegebenen Bedingungen auch das Wasser als nucleophiles Reagens und bildet Phenole. Abgesehen davon, daß dadurch die Ausbeute an Arylestern sinkt, wird auch ihre Reindarstellung erheblich erschwert, so daß sich dieses Verfahren nicht zur technischen Gewinnung von Carbonsäurearylestern eignet.

Außerdem entsteht gemäß der Lehre der US-Patentschrift trotz des Vorhandenseins von Carboxylatanionen im eingesetzten Phenyl-diacetat-thallium (III) bei Abwesenheit anderer Nucleophile in Wasser nur Phenol.

Die vorliegende Erfindung hat die Aufgabe, ein Verfahren für die technische Gewinnung isomerenreiner Carbonsäurearylester zu liefern, bei dem diese Ester einfach und in hoher Ausbeute erhalten werden und sich leicht rein darstellen lassen.

Die gestellte Aufgabe wird erfindungsgemäß durch das in Anspruch 1 beschriebene Verfahren gelöst.

Es wurde nun gefunden, daß grundsätzlich jedes Monoarylthalliumsalz, das nach bekannten Methoden darstellbar ist, für die erfindungsgemäße Umsetzung geeignet ist.

Heteroarylreste können z.B. Thiophene oder Benzofurane sein. Diese Monoarylthalliumsalze bilden Carbonsäurearylester in wäßrigen Lösungsmitteln wie 5-50 %iger Carbonsäure, vorzugsweise jedoch in reinem Wasser, bei Temperaturen zwischen 20 und 100 °C, vorzugsweise bei 40-70 °C, in Gegenwart katalytischer Mengen von Palladiumsalzen, vorzugsweise $PdCl_2$, $Pd(NO_3)_2$, $Pd(ClO_4)_2$ und $Pd(CH_3COO)_2$.

Die US-PS 4,182,915 beschreibt unter anderem auch die Verwendung eines Palladiumsalzes. Jedoch zeigt Tabelle VIIa in Beispiel 8, daß selbst große Mengen $PdCl_2$ bei den Reaktionen dieser Patentschrift nahezu keine katalytische Wirkung haben. Um so überraschender ist es, daß selbst bei den schonenden Bedingungen des erfindungsgemäßen Verfahrens katalytische Mengen an löslichen Palladiumsalzen eine nahezu quantitative Umsetzung bedingen. Die Reaktion kann unter Normaldruck erfolgen. Als Nebenprodukt werden die entsprechenden symmetrischen Biaryle beobachtet. Diese Reaktion wird in konzentrierten Säuren, wie z.B. über 50 %iger Essigsäure, zur Hauptreaktion.

Die nach dem Verfahren darstellbaren Arylester entsprechen hinsichtlich ihres Substitutionsmusters dem eingesetzten Arylthallium(III)-salz, d.h. der Einbau der Carboxylat-Gruppierung erfolgt an der zuvor vom Thalliumrest eingenommenen Position. Die Arylester werden somit isomerenrein dargestellt.

Die Carbonsäurearylester scheiden sich auf der wäßrigen Phase ab und können direkt oder vermittels eines organischen Lösungsmittels — bevorzugt der zu funktionalisierende Ausgangskohlenwasserstoff, falls er einen Siedepunkt nicht wesentlich über 100 °C besitzt, ansonsten vorteilhaft Cyclohexan oder Petroläther — abgetrennt werden und anschließend destillativ oder durch Kristallisation aufgearbeitet werden.

Es wurde gefunden, daß sich als Ausgangsverbindungen besonders solche Monarylthalliumsalze eignen, die die gewünschten Aryl- und Carbonsäurereste enthalten. Die gut darstellbaren Monoarylthalliumdicarboxylate sind beispielsweise für Propionsäure- und Isobuttersäureester geeignete Ausgangsverbindungen. Für die Darstellung der Essigsäureester erwies es sich als vorteilhaft, Monoarylthalliummonoacetatsalze starker Säuren, wie Perchlorsäure und Perfluorbutansulfonsäure, zu verwenden. Diese sind leichter zugänglich als die Diacetate und besitzen gegenüber den in ausgezeichneten Ausbeuten und Reinheiten darstellbaren Bistrifluoracetaten den Vorteil, auf die Verwendung der sehr teuren Trifluoressigsäure zugunsten der wohlfeilen Essigsäure verzichten zu können.

Das erfindungsgemäße Verfahren schließt die Herstellung der bislang unbekannten Monoarylthalliummonoacetatsalze der Perfluorbutansulfonsäure ein. In Eisessig als Lösungsmittel liefern die Reaktionsgemische äquimolarer Mengen Aromat, Perfluorbutansulfonsäure und Thallium(III)-acetat bei Reaktionstemperaturen von 40-110 °C, vorzugsweise bei 70 °C, kristalline

Produkte hoher Isomerenreinheit. Diese Salze sind nach dem Abfiltrieren direkt für Umsetzungen wie oben beschrieben geeignet. Sie besitzten die allgemeine Zusammensetzung $ArTl(CH_3COO)C_4F_9SO_3$ und bilden gemäß den Gesetzmäßigkeiten der Monoarylthalliumsalze mit Alkalijodiden das entsprechende Aryljodid in quantitativer Ausbeute, was neben NMR-spektroskopischen Untersuchungen zur Bestimmung der Isomerenverteilung benutzt werden kann.

Die nach dem Verfahren herstellbaren Carbonsäurearylester besitzen eine Bedeutung als Duftstoffe in der Parfümindustrie, besonders der Essigsäure-p-Tolylester.

Die Essigsäurearylester lassen sich außerdem thermisch in Phenole und Keten überführen. Carbonsäurearylester stellen im übrigen wertvolle Ausgangspunkte für chemische Synthesen dar, wie durch die als Fries-Umlagerung bekannte Darstellung von o- und p-Hydroxiactophenonen aus Essigsäurearylestern belegt ist.

Beispiel 1

In eine Mischung von 10 Gew.-Teilen 5 %iger Essigsäure, die 0,003 Gew.-Teile $PdCl_2$ gelöst enthält, wird portionsweise im Laufe einer Stunde bei 40 °C 1 Gewichtsteil p-Tolylthalliumbis(trifluroacetat) eingetragen und 4 Stunden bei 40 °C gerührt. Nach der Extraktion mit 4 Gew.-Teilen Toluol erhält man 0,17 Gew.-Teile eines Produktgemisches, das aus 44,6 % p-Tolylacetat und 55,4 % 4,4'-Dimethylbiphenyl besteht. Die Gesamtausbeute beträgt 75,1 % der Theorie.

Beispiel 2

Zu einer Lösung von 1 Gew.-Teil p-Tolylthalliumacetat-(perchlorat) in 10 Gew.-Teilen Wasser werden 0,0025 Gew.-Teile $PdCl_2$, gelöst in 2,5 Gew.-Teilen Wasser, gegeben und 2 Stunden bei 60 °C gerührt. Es scheidet sich ein gelbliches Oel ab. Nach Extraktion der Lösung mit Toluol ergeben die vereinigten organischen Phasen 0,216 Gew.-Teile eines Produktgemisches, das aus 86,1 % p-Tolylacetat und 13,9 % 4,4'-Dimethylbiphenyl besteht. Die Gesamtausbeute beträgt 74,0 % der Theorie. Die Reaktionslösung, die nicht umgesetztes Ausgangsprodukt, Thallium(I)-perchlorat und den Katalysator enthält, ist für weitere Umsetzungen verwendbar.

Beispiel 3

1,00 Gew.-Teile Toluol, 4,18 Gew.-Teile Thallium(III)-acetat und 3,49 Gew.-Teile Perfluorbutansulfonsäure werden in 10 Gew.-Teilen Eisessig gelöst und 24 Stunden bei 70 °C gerührt. Beim Abkühlen scheidet sich eine kristalline, farblose Substanz ab, die in Aether mit wenig Aethanol löslich ist und nach Fällung mit Cyclohexan 4,44 Teile isomerenreines p-Tolylthalliumacetat (perfluorbutansulfonat) liefert (Ausbeute 62 % der Theorie).

$C_{13}H_{10}F_9O_5STl$ (653,56)

| | | | | | |
|---|---|---|---|---|---|
| Ber. | C 23,89 | H 1,54 | F 26,16 | S 4,90 | Tl 31,27 |
| Gef. | C 23,54 | H 1,71 | F 25,82 | S 5,01 | Tl 31,00 |

Beispiel 4

In 10 Gew.-Teile Wasser, das 0,002 Gew.-Teile $PdCl_2$ gelöst enthält, wird portionsweise im Laufe einer halben Stunde bei 60 °C 1 Gew.-Teil p-Tolylthalliumacetat(perfluorbutansulfonat) (hergestellt entspr. Beispiel 3) eingetragen und 4 Stunden bei 60 °C gerührt. Nach der Extraktion mit 4 Gew.-Teilen Toluol erhält man 0,146 Gew.-Teile eines Produktgemisches, das aus 80,8 % p-Tolylacetat und 19,2 % 4,4'-Dimethylbiphenyl besteht. Die Gesamtausbeute beträgt 71,4 % der Theorie. Die Reaktionslösung, die neben unumgesetztem Einsatzprodukt und Thallium(I)-perfluorbutansulfonat noch den Katalysator enthält, ist für weitere Umsetzungen verwendbar.

Vergleichsbeispiel

10 g (21,2 mMol) p-Tolylthalliumacetatperchlorat-Hydrat und 4,23 g (21,2 mMol) Kupfer(II)-acetat-Hydrat wurden in 100 g Wasser 5 Stunden lang bei 60 °C gerührt. Anschließend wurde mit 50 g Benzol extrahiert und die organische Phase der GC-Analyse unterworfen. Weder p-Tolylacetat noch p-Kresol waren nachweisbar.

**Ansprüche**

1. Verfahren zur Herstellung isomerenreiner Arylester von Carbonsäuren, dadurch gekennzeichnet, daß Monoarylthallium (III) salze der allgemeinen Formel

$$Ar—TlXY,$$

worin Ar sowohl ein- oder mehrkernige Arylreste als auch ein- oder mehrkernige Heteroarylreste, X und Y sowohl identische Carboxylatreste als auch verschiedene Carboxylatreste oder X einen Carboxylatrest und Y einen Säurerest einer starken Säure bedeuten, in reinem Wasser oder einem wäßrigen Lösungsmittel in Gegenwart katalytischer Mengen von Palladiumsalzen unter milden Bedingungen zersetzt und die erhaltenen Arylester von der wäßrigen Phase in bekannter Weise abgeschieden oder mit Lösungsmitteln extrahiert werden.

2. Verfahren nach Anspruch 1, dadurch gekennzeichnet, daß die Zersetzung in reinem Wasser durchgeführt wird.

3. Verfahren nach Anspruch 1, dadurch gekennzeichnet, daß die Zersetzung in 5-50 %iger wasserlöslicher Carbonsäure durchgeführt wird.

4. Verfahren nach den Ansprüchen 1 und 2, dadurch gekennzeichnet, daß als Monoarylthalliumsalze solche der Perfluorbutansulfonsäure mit der allgemeinen Formel

$$Ar—TlX\ C_4F_9SO_3$$

zur Reaktion gebracht werden.

5. Verfahren nach den Ansprüchen 1 und 2, dadurch gekennzeichnet, daß man als Monoarylthalliumsalze solche der allgemeinen Formel

$$Ar—TlX\ ClO_4$$

zur Reaktion bringt.

6. Verfahren nach den Ansprüchen 1 bis 6, dadurch gekennzeichnet, daß die einzelnen Komponenten bei 40-70 °C unter normalem Druck innerhalb 2-4 Stunden, gegebenenfalls unter Rühren, zur Reaktion gebracht werden und die Reaktionsprodukte in an sich bekannter Weise abgetrennt werden.

**Claims**

1. A process for the preparation of aryl esters of organic carboxylic acids in isomeric purity comprising decomposing a compound of the general formula

$$Ar—TlXY$$

wherein Ar is a mono- or polycyclic aryl radical or a mono- or polycyclic heteroaryl radical and X and Y are either identical or different acyloxy radicals or organic carboxylic acids or X is an acyloxy radical of an organic carboxylic acid and Y is an anion of a strong mineral acid, in pure water or in an aqueous solvent in the presence of catalytic amounts of palladium salts under mild conditions and separating the resulting aryl esters in a known manner from the aqueous phase or extracting them with solvents.

2. The process of claim 1 wherein the decomposition is made in pure water.

3. The process of claim 1 wherein the decomposition is made in a 5 to 50% watersoluble carboxylic acid.

4. The process of claims 1 and 2 wherein as monoaryl thallium salts, salts of the perfluorobutane sulfonic acid of the general formula

$$Ar—TlX\ C_4F_9SO_3$$

are decomposed.

5. The process of claims 1 and 2 wherein as monoaryl thallium salts, salts of the general formula

$$Ar—TlX\ ClO_4$$

are decomposed.

6. The process of claims 1 to 5 wherein the components are reacted at temperatures of 40 to 70 °C under atmospheric pressure within 2 to 4 hours optionally under stirring and the reaction products are separated in a known manner.

**Revendications**

1. Procédé de préparation d'esters aryliques d'acides carboxyliques à l'état d'isomères purs caractérisé en ce que l'on décompose des sels de monoarylthallium-III de formule générale

$$Ar—TlXY,$$

dans laquelle Ar représente des restes aryles mono- ou polycycliques ou des restes hétéroaryles mono- ou polycycliques, X et Y représentent des restes carboxylates identiques ou différents ou bien X représente un reste de carboxylate et Y un reste acide d'acide fort, dans l'eau pure ou dans un solvant aqueux, en présence de quantités catalytiques de sels de palladium, dans des conditions ménagées, et on sépare les esters aryliques obtenus de la phase aqueuse ou on les extrait par des solvants de manière connue en soi.

2. Procédé selon la revendication 1, caractérisé en ce que la décomposition est effectuée dans l'eau pure.

3. Procédé selon la revendication 1, caractérisé en ce que la décomposition est effectuée dans un acide carboxylique hydrosoluble à une concentration de 5 à 50%.

4. Procédé selon les revendications 1 et 2, caractérisé en ce que l'on fait réagir en tant que sels de monoaryl-thallium les sels de l'acide perfluorobutanesulfonique de formule générale

$$Ar—TlX\ C_4F_9SO_3.$$

5. Procédé selon les revendications 1 et 2, caractérisé en ce que l'on fait réagir en tant que sels de monoaryl-thallium les sels répondant à la formule générale

$$Ar—TlX\ ClO_4.$$

6. Procédé selon les revendications 1 à 6, caractérisé en ce que l'on fait réagir les composants individuels à une température de 40 à 70 °C, sous pression normale, en 2 à 4 heures, éventuellement sous agitation et on sépare les produits de réaction de manière connue en soi.